# EUROPEAN PATENT APPLICATION

(11) **EP 3 599 281 A1**
(43) Date of publication of application: **29.01.2020**
(21) Application number: 18185845.7
(22) Date of filing: 26.07.2018
(51) Int. Cl.: C12P 13/00, C12N 9/04

(54) **PROCESS FOR THE MANUFACTURE OF NITRILE COMPOUNDS**

(71) Applicant: Universiteit van Amsterdam, 1012 WX Amsterdam (NL)
(72) Inventor: MUTTI, Francesco, 1098 XH Amsterdam (NL); VILIM, Jan, 1098 XH Amsterdam (NL); KNAUS, Tanja, 1098 XH Amsterdam (NL)
(74) Representative: De Vries & Metman

(57) **Abstract**

The invention pertains to a process for manufacturing aromatic nitriles, wherein a starting material selected from the group of aromatic primary alcohols and aromatic aldehydes is contacted under reaction conditions in a reaction vessel with an inorganic amine compound and molecular oxygen in the presence of a Cu-dependent alcohol oxidase.

The process of the invention makes it possible to prepare aromatic nitriles from easily available starting materials, namely aromatic primary alcohols or aldehydes, in high efficiency, without having to rely on cyanides or azides, or stringent reaction conditions.

## Description

The present invention pertains to a process for manufacturing nitrile compounds.

Nitrile compounds, in particular aromatic nitrile compounds are of interest for a number of industrial applications. For example, benzyl nitrile, and derivatives thereof, constitute an important active core, which is present in an increasing number of pharmaceuticals. Only in 2010, over 30 nitrile-containing pharmaceuticals were prescribed for a diverse variety of medicinal indications with more than 20 additional nitrile-containing leads in clinical development. (F. F. Fleming et al. J. Med. Chem., 2010, 53, 7902-7917). Cinnamyl nitrile is used in industry as a fragrance. Nicotinonitrile (also called 3-pyridinecarbonitrile or 3-cyanopyridine) is also of industrial interest. For instance, it is the precursor for the production of nicotinamide (vitamine B3). It is also used as agent for capturing water in heterogeneous catalysis processes.

In the art, nitriles have been synthesised in various manners. However, many of them rely on the use of highly toxic starting materials such as cyanide compounds or azides.

For example the review publication by Beller's group (Anbarasan et al. Chem. Soc. Rev., 2011, 40, 5049-5067) describes a few processes wherein substituted aryl halides are reacted with a cyanide salt (e.g. potassium cyanide, copper cyanide) to form substituted benzonitriles.

Cyanide-free routes starting from aldehydes require ammonium salts or toxic azide or hydroxylamine. All these routes involve the additional use of ungreen organic solvents (DMSO, acetonitrile) and/or expensive stoichiometric oxidants and/or catalytic TEMPO and/or metal-catalyst in elevated amount and/or additional additives (e.g. tri-ethylamine) and/or strong acids. For example, an article by Noh and Kim (Noh et al. J. Org. Chem. 2015, 80, 11624-11628) describes the conversion of substituted benzaldehydes into the corresponding substituted benzonitriles by reacting benzaldehyde with a large excess of ammonium acetate (2.4 equivalents) in presence of TEMPO (5 mol%), sodium nitrite (10 mol%), nitric acid (20 mol%) in acetic acid at 50°C. In another example (Rokade et al. J. Org. Chem. 2012, 77, 5364-5370), it is described that substituted benzaldehydes are reacted with sodium azide (1.5 equivalents), triflic acid (3 equivalents) in acetonitrile. In another example (Augustine et al. Synlett 2011, 15, 2223-2227), it is described that substituted benzaldehydes are reacted with hydroxylamine hydrochloride (1.1 equivalent) in pure DMSO at 90 °C. Cyanide-free routes from alcohols are even more challenging in chemistry. They require metal and/or an organic catalyst in presence of supra-stoichiometric amounts of an organic oxidant. The requirement for organic oxidant reduces considerably the atom-efficiency because of the generation of supra-stoichiometric amount of waste. In addition, most of these methods require elevated catalyst loading and/or concentrated ammonia and/or additional heat. For example, an article by Vatéle (Vatéle, Synlett 2014, 25, 1275-1278) describes a process wherein substituted benzylic alcohols are reacted with TEMPO (5 mol%) as organocatalyst, Phl(OAc)₂ (2.2 equivalents) as organic oxidant, ammonium salt as nitrogen source (4 equivalents) in acetonitrile-water (9:1 in volume) or dichloromethane-water (9:1 in volume) as solvent.

Routes starting out from alcohols and consuming molecular oxygen (from air) as ultimate oxidant have also been described. However, they also suffer from disadvantages.

A publication by Bhanage's group (Yadav et al. Eur. J. Org. Chem. 2013, 5106-5110) describes the conversion of benzyl alcohol derivatives to benzonitrile derivatives using copper (II) chloride (10 mol%), potassium carbonate (10 mol%), ammonium formate (2-4 equivalents) in solid phase, with additional air as oxidant. This method relies on elevated amounts of copper and potassium salts, which generates waste, and relatively high temperatures (135°C).

A publication by Batra's group (Dighe et al. Adv. Synth. Catal. 2014, 356, 3892-3896) describes the conversion of benzyl alcohol to benzonitrile using iron (III) chloride (5 mol%) as catalyst in presence of TEMPO as cocatalyst (at least 5 mol%) and aqueous ammonia, in organic solvent (e.g. acetonitrile) and air. This method requires relatively large amounts of a mixture of catalyst and cocatalyst amount along with low turnover numbers (max calculated TON 15-20) and the use of an organic solvent.

Enzymatic methods for the synthesis of nitriles have also been described. These methods include the use of aldoxime dehydratases, hydroxynitrile lyases (i.e. addition of cyanide to carbonyl compounds), halohydrin dehalogenases (i.e. ring-opening of epoxides by cyanide), and amine oxidases in combination with cyanide salt. However, many of these enzymatic routes rely on the use of toxic cyanide (e.g., as described in Zheng et al. Adv. Synth. Catal. 2017, 359, 1185-1193 or in Schallmey et. al. Appl. Microbiol. Biotechnol. 2016, 100, 7827-7839 or in Kawahara et al. Green Chem. 2017, 19, 418-424) or exotic starting materials such as the aldoxime used in Metzner et al. ChemCatChem 2014, 6, 3105-3109.

There is need in the art for a method for manufacturing aromatic nitriles from easily available starting materials, in high efficiency, without having to rely on cyanides or azides, or stringent reaction conditions. The present invention provides such a process.

The invention pertains to a process for manufacturing aromatic nitriles, wherein a starting material selected from the group of aromatic primary alcohols and aromatic aldehydes is contacted under reaction conditions in a reaction vessel with an inorganic amine compound and oxygen in the presence of a Cu-dependent alcohol oxidase.

In nature, alcohol oxidases perform the oxidation of primary alcohols to aldehydes and secondary alcohols to ketones. Surprisingly it has been found that in presence of an inorganic nitrogen source in aqueous solution, Cu-dependent alcohol oxidases can catalyze a subsequent oxidation of the aldehyde to give nitrile as the final product. This discovery makes it possible to prepare aromatic nitriles from easily available starting materials, namely aromatic primary alcohols or aldehydes, in high efficiency, without having to rely on cyanides or azides, or stringent reaction conditions.

The process according to the invention and the advantages associated therewith will be elucidated further below.

The starting material in the process according to the invention is selected from the group of aromatic primary alcohols and aromatic aldehydes.

Aromatic primary alcohols suitable for use in the process according to the invention are compounds of the formula Ar-R-CH₂OH, wherein Ar is an aryl group, and R is a covalent bond or a C1-C20 alkylene moiety.

Ar stands for an aryl group, which may be a hetero-aryl group (i.e. the aromatic structure comprising one or more atoms selected from O, S, or N), or which may be a non-heteroarylgroup. The aryl group may be unsubstituted or substituted with one or more alkyl, substituted alkyl, nitro, alkoxy, or halo groups. Alkyl groups and alkoxy groups present as substituent of the Ar group generally have 1-10 carbon atoms. "substituted alkyl" means an alkyl group having a substituent containing a heteroatom or heteroatoms such as N, O or S; "halo" means F, Cl, Br, or I. The arylgroup may be a single-ring structure or a conjugated ring structure. Examples of suitable aryl groups are
- substituted or unsubstituted phenyl
- substituted or unsubstituted naphthalyl

Examples of suitable heteroaryl groups are:
- substituted or unsubstituted furanyl
- substituted or unsubstituted pyridinyl
- substituted or unsubstituted pyrazinyl
- substituted or unsubstituted pyrolyl
- substituted or unsubstituted imidazolyl
- substituted or unsubstituted pyrazolyl, and
- substituted or unsubstituted oxazolyl.

In a preferred embodiment, Ar is a phenyl group, optionally substituted with one or more of halogen, in particular Cl or Br, a C1-C4 alkyl group, or a C1-C4 alkoxy group. In another preferred embodiment, Ar is a pyridinyl group, optionally substituted with one or more of halogen, in particular Cl or Br, a C1-C4 alkyl group, or a C1-C4 alkoxy group. The use of unsubstituted phenyl or unsubstituted pyridinyl may be particularly preferred.

In the formula Ar-R-CH₂OH R stands for a covalent bond or an alkylene moiety with 1-20 carbon atoms, in particular 1-10 carbon atoms. The alkylene moiety group may be straight-chain or branched, saturated or unsaturated, and unsubstituted or substituted with one or more alkyl, substituted alkyl, nitro, alkoxy, or halo groups. It may be preferred for R to be a covalent bond or a straight-chain alkylene moiety with 1-4 carbon atoms or a straight-chain alkylene moiety with 2-5 carbon atoms at least one double bond. It may be particularly preferred for R to be a covalent bond or a -CH=CH- moiety.

Preferred aromatic primary alcohols suitable for use in the process according to the invention include benzylalcohol, o-, m-, or p-chlorobenzylalcohol, o-, m-, or p-bromobenzylalcohol, o-, m-, or p-fluorobenzylalcohol, o-, m-, or p-methylbenzylalcohol, o-, m-, or p-methoxybenzylalcohol, and dimethoxybenzylalcohol, in particular benzylalcohol.

Another preferred aromatic primary alcohol for use in the present invention encompasses cinnamylalcohol. Preferred heteroaromatic primary alcohols include 3-(hydroxymethyl)pyridine and 4-(hydroxymethyl)pyridine.

Aromatic aldehydes can also be used as starting materials in the process according to the invention. Suitable compounds include compounds of the formula Ar-R-COH wherein Ar is an aryl group, and R is a covalent bond or an alkylene moiety. Ar and R are as defined above.

Preferred aldehydes suitable for use as starting material in the present invention include benzylaldehye, o-, m-, or p-chlorobenzylaldehyde, o-, m-, or p-bromobenzylaldehyde, o-, m-, or p-fluorobenzylaldehyde, o-, m-, or p-methylbenzylaldehyde, o-, m-, or p-methoxybenzylaldehyde, dimethoxybenzylaldehyde, and cinnamylaldehyde.

In general, the use of aromatic primary alcohols as starting materials may be preferred, as these compounds may be more readily available than aromatic aldehydes.

The enzyme used in the present invention is a Cu-dependent alcohol oxidase. Cu-dependent alcohol oxidases are known in the art.

In one embodiment, the enzyme comprises the aminoacid sequence SEQ ID NO1: 
ASAPIGSAIPRNNWAVTCDSAQSGNECNKAIDGNKDTFWHTFYGANGDPKPPHTYTIDMKTTQNVNGL
SVLPRQDGNQNGWIGRHEVYLSSDGTNWGSPVASGSWFADSTTKYSNFETRPARYVRLVAITEANGQP
WTSIAEINVFQASSYTAPQPGLGRWGPTIDLPIVPAAAAIEPTSGRVLMWSSYRNDAFEGSPGGITLT
SSWDPSTGIVSDRTVTVTKHDMFCPGISMDGNGQIVVTGGNDAKKTSLYDSSSDSWIPGPDMQVARGY
QSSATMSDGRVFTIGGSFSGGVFEKNGEVYSPSSKTWTSLPNAKVNPMLTADKQGLYMSDNHAWLFGW
KKGSVFQAGPSTAMNWYYTSGSGDVKSAGKRQSNRGVAPDAMCGNAVMYDAVKGKILTFGGSPDYTDS
DATTNAHIITLGEPGTSPNTVFASNGLYFARTFHTSVVLPDGSTFITGGQRRGIPFEDSTPVFTPEIY
VPEQDTFYKQNPNSIVRAYHSISLLLPDGRVFNGGGGLCGDCTTNHFDAQIFTPNYLYDSNGNLATRP
KITRTSTQSVKVGGRITISTDSSISKASLIRYGTATHTVNTDQRRIPLTLTNNGGNSYSFQVPSDSGV
ALPGYWMLFVMNSAGVPSVASTIRVTQ
 or a sequence having at least 60% sequence identity with SEQ ID NO1, in particular at least 70%, more in particular at least 80%, still more in particular at least 90%. In one embodiment, the enzyme has the aminoacid sequence SEQ ID NO1.

An enzyme comprising the above sequence is described in Escalettes et al., Directed Evolution of Galactose Oxidase: Generation of Enantioselective Secondary Alcohol Oxidases, ChemBioChem, 2008, 9, 857-860, as a variant of the Cu-dependent galactose oxidase derived from Fusarium sp.

In general, any enzyme showing a similar active site as the one herein described will be able to catalyze the reaction described in this invention. Accordingly, in one embodiment, an enzyme is used which comprises an active centre equivalent to the active centre of the enzyme comprising or having the aminoacid sequence above. This will be elucidated below. The coordination sphere of the active centre of the enzyme is described in Whittaker, J.W., The radical chemistry of galactose oxidase. Arch Biochem Biophys, 2005. 433(1): p. 227-239. Cu-dependent alcohol oxidases contain a catalytic copper cation in the active site, which changes oxidation state from +1 to +2 during the catalytic cycle. The copper cation is in general coordinated to four amino acid residues of the active site: Tyrosine-272, Histidine-581, Histidine-496 and Tyrosine-495. These aminoacids are indicated in **bold** in the sequence above (Y-272; Y-495; H-496; H-581). Amino acid residue Tyrosine-272 forms a peculiar cross-link with Cysteine-228, generating a Tyrosine-Cysteine adduct. This crosslink is fundamental for catalysis as it allows for the stabilization of a free radical species in the active site of the enzyme (Whittaker, 2005). In practice, the cooperativity between the copper +1/+2 metal centre and the radical Tyrosine-272 enables the simultaneous transfer of two electrons from the substrate to the enzyme. Another important amino acid residue in the active site of the GOx enzyme used in this invention is the Phenylalanine-290. We conclude that the amino acid residue Phenylalanine-290 is also crucial for enzymatic activity towards the synthesis of aromatic nitriles in this invention.

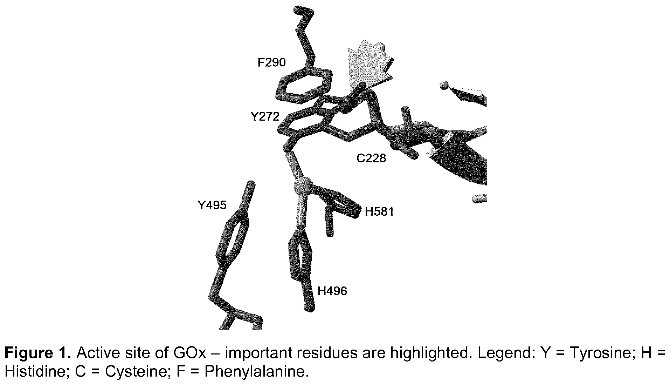

The important residues for catalytic activity are highlighted in Figure 1 (derived from Whittaker, J.W., The radical chemistry of galactose oxidase. Arch Biochem Biophys, 2005. 433(1): p. 227-239,.Yin et al., Structure-function characterization reveals new catalytic diversity in the galactose oxidase and glyoxal oxidase family. Nature Communications, 2015. 6 (10197)).

In general, any enzyme showing a similar active site as the one herein described will be able to catalyze the reaction described in this invention. Enzymes having a similar active site as the one herein described are Cu-dependent enzymes containing a Cu cation in the active site, wherein the active site is formed by a Tyrosine residue corresponding to Tyr272, a Histidine residue corresponding to Hys581, a Histidine residue corresponding to His496, a Tyrosine residue corresponding to Tyr495, and a Phenylalanine residue corresponding to Phe290, wherein the Tyrosine residue corresponding to Tyr272 is crosslinked with a Cysteine residue corresponding to Cys228, thereby generating a Tyr-Cys adduct, wherein the Cu cation is coordinated to the amino acid residues of the active site, and wherein Tyr272, Hys581, His496, Tyr495, Phe290, and Cys228 resemble the amino acid residues and their positions in the amino acid sequence given above. It will be clear that it is not required for the enzymes which are capable to catalyse the reaction described in this invention to have the amino acid sequence given above, as long as the amino acid sequence of these enzymes allows the formation of the active site required to coordinate the Cu cation and to catalyze the reaction of the present invention.

The enzyme can be applied in purified form. It can also be applied in the form of a cell-free enzyme extract (CFE). The latter embodiment is preferred, because the preparation of CFEs requires less purification steps, which is attractive from an economic point of view.

The reaction is carried out in a liquid reaction medium which comprises the starting material, the enzyme, the inorganic amine compound, molecular oxygen, and optional further components.

The reaction is carried out in the presence of an inorganic amine compound, which provides the nitrogen to form the nitrile. The inorganic amine compound is selected from ammonia (NH₃) and the ammonium ion (NH₄⁺). Both compounds have been found to result in a high nitrile yield.

Ammonia (NH₃) can be provided to the reaction mechanism in the form of a gas, e.g., by bubbling through the reaction mixture. It can also be provided in the form of an aqueous solution (which strictly speaking will encompass the provision of the ammonia as ammonium ion). If the reaction is carried out in an aqueous medium, the ammonia will convert to ammonium in the reaction mixture, also when it is provided in gaseous form.

Ammonium can easily be provided in the form of an ammonium salt, as ammonium salts can have a high solubility in water. In one embodiment, the ammonium ion is provided in the form of a salt of an inorganic or organic acid. The anion of the salt should be selected such that it does not interfere with the reaction. Suitable anions include anions of organic acids, e.g., formate, lactate, citrate, gluconate, tartrate, etc. Suitable inorganic anions include nitrate, sulphate, phosphate, chloride, and chlorate.

As the inorganic amine compound is a reactant in the process according to the invention, it is preferred for the molar ratio of nitrogen source to starting compound to should be at least 0.5:1, in particular at least 1:1.

Where the inorganic amine compound is an ammonium ion, it can be present in a large excess, e.g., where the reaction is carried out in a buffer based on an ammonium salt of a weak acid.

While there is no upper limit on the absolute amount of inorganic amine compound present in the system as long as physical saturation is reached, it may be preferred to keep the concentration from becoming too high, because very high amounts of inorganic amine compound may affect the enzyme. In one embodiment, the reaction medium has an ammonium concentration of at most 1.2 M, more in particular at most 1.0M, still more in particular at most 0.80 M. As a minimum concentration an ammonium concentration of 10 mM may be mentioned, e.g., at least 50 mM, or at least 0.1M.

The reaction is carried out in the presence of (molecular) oxygen (O₂). Oxygen can be provided simply by carrying out the reaction in an oxygen-containing atmosphere, such as air. If so desired, additional oxygen can be provided, which may lead to an increase in conversion. Methods for providing additional oxygen, and for ensuring that the contact between the gaseous oxygen-containing environment and the reaction medium is improved are known in the art and require no further elucidation. They include, e.g., stirring the reaction medium, bubbling gas through the reaction medium, etc.

The reaction takes place in a liquid medium, which may be aqueous (that is, water is the main solvent present in the system), or non-aqueous (that is, a solvent or combination of solvent other than water is/are the main solvent present in the system). A solvent is defined as a compound or combination of compounds which provides the liquid phase of the reaction medium. A main solvent is a solvent or combination of solvents which makes up more than 50 vol.% of the liquid phase of the reaction medium.

Where the reaction medium is a non-aqueous reaction medium, suitable solvents include alcohols, esters, ethers, alkanes, sulfoxides, amides, simple nitriles etc., for example, ethylacetate, methyl tert-butyl ether, octane, and toluene, DMSO, DMF, acetonitrile. Where the reaction medium is a non-aqueous reaction medium it may be preferred to use relatively hydrophobic solvents, such as ethers, esters, and alkanes, as this will result in retention of higher enzyme activity and stability in comparison to hydrophilic solvents. In this case, the enzyme will generally be dispersed in solid form in the reaction medium, e.g. in lyophilized form or immobilized on a carrier material. In this case it is preferred for the inorganic amine compound to be ammonia which is provided in the form of a gas. The starting compound can be dissolved in the reaction medium. Biocatalysis in non-aqueous reaction media is known in the art. Reference is made, e.g., to "Organic Synthesis with Enzymes in Non-aqueous media", edited by G. Carrea and S. Riva, 2008, Wiley-VCH Verlag GmbH & Co., Weinheim (Germany).

Performance of the reaction in an aqueous reaction medium may be preferred, as this will allow dissolving the inorganic amine compound and the enzyme in the same reaction phase (i.e. water) giving, for instance, improved mass transfer. As some of the starting compounds have a limited solubility in water, it may be attractive to provide them dissolved in an organic solvent with a high miscibility with water when the reaction is performed in an aqueous medium, e.g., DMSO or lower alcohols such as ethanol and methanol.

Reaction conditions include a suitable reaction temperature. The reaction temperature generally is between 5°C and 65°C, in particular between 10°C and 60°C. In one embodiment the reaction temperature is between 10°C and 50°C, in particular between 10 °C and 40°C. In some embodiments, depending on the enzyme used, the temperature is between 15°C and 30°C.

Reactions pressure is not critical to the process according to the invention. Any pressure between 0.5 and 3 atmosphere will suffice. Atmospheric pressure is suitable and attractive from an economic point of view.

In the present invention, the reaction is generally carried out at a pH of 7-12. It may be preferred for the pH to be between 8 and 11, more in particular between 8 and 10. Depending on the nature of the enzyme, the pH may attractively be between 8.5 and 9.5.

In one embodiment, the reaction is carried out in a buffer. Buffers are conventionally used to keep the pH of a reaction medium at a certain value, e.g., a value where the enzyme shows an optimum. In the present case, the use of ammonium-based buffers may be particularly attractive, because an ammonium buffer will also provide ammonium as nitrogen source. Examples of suitable buffers are ammonium formate, ammonium chloride, ammonium acetate, ammonium chlorate, ammonium phosphate, and ammonium sulphate.

The enzyme used in the present invention is a Cu-dependent oxidase. As will appear from the discussion above, a Cu-ion is an essential part of the active site of the enzyme. When the enzyme is provided to the reaction medium, Cu is automatically provided with the enzyme. However, as the metal cofactor may be not tightly bound when the enzyme is applied in solution, it may be attractive to produce additional Cu²⁺ to the reaction medium. Cu²⁺ can suitable be provided through a solution of a copper(II) salt. The nature of the salt is not critical, as long as the anion does not interfere with the reaction and as long as the copper salt is soluble in the reaction medium. Suitable salts include inorganic copper(II) salts such as copper sulphate, copper nitrate, copper bromide, copper chlorate, and copper chloride, and organic copper(II) salts such as copper formate, copper tartrate, and copper oxalate.

If Cu²⁺ is added to the reaction medium, the molar ratio of Cu to enzyme will generally be at least 0.2:1 to make it worth the effort. More in particular it may be at least 0.5:1, more specifically at least 1:1. Much higher ratios may be used, which may lead to increased conversion, e.g., at least 5;1 at least 10:1, or at least 20:1, or at least 40:1. As a general maximum a value of at most 200:1 may be mentioned, while 150:1 may also be a suitable maximum.

Many Cu-dependent oxidases produce H₂O₂ as a secondary product. H₂O₂ may detrimentally affect the activity of the enzyme. Therefore, it may be attractive to remove the H₂O₂ from the reaction medium during the reaction.

One way of removing H₂O₂ from the reaction medium is by the addition of a catalase, which converts the H₂O₂ into water and molecular oxygen. If used, catalase may be added in an amount of at least 1 mM per mole Cu-dependent oxidase enzyme, in particular at least 0.1 mole per mole Cu-dependent oxidase enzyme. As a maximum value an amount of at most 10 mole per mole Cu-dependent oxidase enzyme may be mentioned. Large amounts of catalase have been found to be not necessary. Therefore, it may be preferred to add the catalase in an amount of at most 5 mole per mole Cu-dependent oxidase enzyme. Thus, in one embodiment, catalase will be added in an amount of 0 to 5 mole per mole Cu-dependent oxidase enzyme.

H₂O₂ can also be removed using other enzymes, in particular peroxidases such as horseradish peroxidase or peroxygenase. They can be added in amounts analogous to those given above for catalase. The use of catalase is preferred, however, as it forms only water and molecular oxygen. The addition of chemical H₂O₂ catchers such as water-soluble iron porphyrins can also be considered.

The reaction medium comprising the starting compound selected from the group of aromatic primary alcohols and aromatic aldehydes, the inorganic amine compound, and the Cu-dependent alcohol oxidase can be kept under reaction conditions until the desired degree of conversion is obtained. Generally, the reaction time is at least 30 minutes, more in particular at least 1 hour. In general, keeping the reaction medium under reaction conditions for more than 48 hours will not lead to significant additional conversion. Accordingly, the reaction medium will generally be kept under reaction for 30 minutes to 48 hours, in particular 1 hour to 36 hours. If so desired, the reaction can be actively stopped by the addition of a strong inorganic base, e.g., KOH, but this is not required. As will be evident to the skilled person, care should be taken that the addition of strong inorganic base does not affect the reaction product.

Once the reaction has been completed, the nitrile product can be recovered from the reaction medium by methods known in the art. For example, the nitrile product can be recovered from the reaction medium by extraction with a suitable solvent. Examples of suitable solvents include esters such as ethylacetate and isopropylacetate, ethers such as methyl-tert-butylether, and tetrahydrofurane, and hydrocarbons such as heptane, hexane, and turpentine. Although less attractive from an environmental perspective, solvents such as chloroform, dichloromethane, benzene, and toluene may also be used. It is within the scope of the skilled person to determine a suitable extractant.

Other separation methods can also be used, e.g., absorption onto a solid hydrophobic carrier followed by elution therefrom, chromatography, and, depending on the composition of the reaction medium, distillation.

The product can be subjected to purification methods known in the art, which require no further elucidation here. One of the advantages of the process according to the invention is that the reaction shows a high yield of the desired nitrile product, without substantial formation of side products. In consequence, further purification steps may in general be relatively limited, or not required at all.

The invention will be illustrated by the following examples, without being limited thereto or thereby.

### Examples

### Enzyme preparation

In the examples, galactose oxidase from Fusarium sp. variant M3-5 is used as enzyme, further indicated as GOx. The enzyme is obtained as follows.

### Cloning and expression

By a variation on the process described in F. Escalettes, N. J. Turner, Chembiochem : a European journal of chemical biology 2008, 9, 857-860 and A. Toftgaard Pedersen, W. R. Birmingham, G. Rehn, S. J. Charnock, N. J. Turner, J. M. Woodley, Organic Process Research & Development 2015, 19, 1580-1589, the gene coding for GOx with C-terminal Strep-tag was cloned into the target vector (pET42a), transformed into *E. coli* XL1 Blue and spread on LB agar plate containing Kanamycin (50 mg L⁻¹). Three single clone colonies were picked and used to inoculate overnight cultures (37°C, orbital agitation 170 rpm). Cells were harvested, plasmids were extracted with Roti-prep gel extraction kit (Carl Roth) and the presence of the gene in plasmid was verified by restriction analysis and sequencing (Eurofins). The constructs were cloned into expression strain (BL21 DE3). For recombinant expression of the GOx, LB medium (800 ml) with Kanamycin (50 mg L⁻¹) was inoculated with an overnight culture of E. coli cells (15 ml) harboring expression plasmid with the GOx. Culture was grown at 37 °C until an OD₆₀₀ reached value of 0.6 - 0.9. At that point, the media was supplemented with CuSO₄.5H₂O (cofactor for GOx, 250 mg l⁻¹ final concentration) and subsequently expression was induced by the addition of IPTG (0.5 mM final concentration). Expression was carried out overnight and after harvesting of the cells (4 °C, 3400 g, 15 min), the cell pellet was washed with NaCl solution (0.9%), centrifuged at the same conditions and frozen at -20°C. Expression level was checked by SDS-PAGE of the pre- and after-incubation samples. Obtained cells were used for purification of the protein with affinity chromatography or for the preparation of cell free extract (CFE).

### Enzyme purification

The enzyme was purified by Strep-tag affinity chromatography as follows: Frozen cell pellets containing GOx were thawed, re-suspended in binding buffer (100 mM Tris-HCI, 150 mM NaCl, pH 8) and subsequently incubated with lysozyme (1 mg ml⁻¹) for 30 min at 4°C. The cells dispersion was subsequently sonicated for 15 minutes (10 s on, 15 s off, 45% amp) until reduction of viscosity was observed. Sonicated sample was then centrifuged for 1 hour (ca. 35000 g, 4°C). Supernatant was collected, filtrated through 0.45 µm filter (Whatman) and loaded onto StrepTrapHP (GE Healthcare) according to the purification manual. Purified protein fractions were pooled and dialyzed overnight at 4°C against the binding buffer with six-fold molar excess of Cu⁺² ions compared to the enzyme, to ensure the loading of the active site of the enzyme with Cu²⁺. The next day, the dialysis was repeated against the binding buffer to remove excess of copper. After 24 hours, the enzyme was concentrated using Vivaspin (Milipore), the concentration was determined using the Biorad protein assay kit (BioRad) and the enzyme was flash frozen in liquid nitrogen in 1 ml aliquots. Purity of the enzyme was assessed by SDS-PAGE.

The GOx is present in a Tris-HCI buffer (100 mM, pH 8). In the experiments below, buffer exchange to the reaction buffer was performed using Vivaspin (Milipore), followed by washing with 5 volumes of reaction buffer.

### Analytics

GC-FID was carried out using Agilent J&W DB1701 (30 m, 250 µm, 0.25 µm) and Agilent J&W HP-5 (30 m, 320 µm, 0.25 µm) colums and H2 as carrier gas using one of the following methods.
**Method A:** T injector 250 °C; constant pressure 6.9 psi; temperature program: 80 °C, hold 6.5 min; 10 °C min⁻¹ to 160 °C, hold 5 min; 20 °C min⁻¹ to 200 °C, hold 2 min; 20 °C min⁻¹ to 280 °C, hold 1 min.
**Method B:** injector 250 °C; constant pressure 4.0 psi; temperature program: 60 °C, hold 0 min; 10 °C min⁻¹ to 300 °C, hold 1 min.
**Method C:** T injector 250 °C; constant flow 1.3 ml min⁻¹; temperature program: 60 °C, hold 0 min; 10 °C min⁻¹ to 120 °C, hold 30 min; 10 °C min⁻¹ to 300 °C, hold 1 min.

Retention times of reference compounds are listed in the table below together with the instrument and method used for analysis.

Retention time of IS (toluene) - 3.0 min; retention time of co-solvent (DMSO) - 7.3 min.

List of retention times, type of column and acquisition method used for GC analysis.

| Entry | Substrate | Column | Method | Split ratio | retention time [min] | | |
|---|---|---|---|---|---|---|---|
| | | | | | Alcohol (a) | Aldehyde (b) | Nitrile (c) |
| **1** | Benzyl alcohol | DB1701-30m | Method A | 20 | 11.5 | 8.5 | 9.8 |
| **2** | 4'-Fluorobenzyl alcohol | DB1701-30m | Method A | 20 | 12.2 | 8.2 | 9.6 |
| **3** | 3'-Fluorobenzyl alcohol | DB1701-30m | Method A | 20 | 12.5 | 8.0 | 8.7 |
| **4** | 2'-Flourobenzyl alcohol | DB1701-30m | Method A | 20 | 11.7 | 7.7 | 10.5 |
| **5** | 4'-Chlorobenzyl alcohol | DB1701-30m | Method A | 20 | 16.1 | 12.7 | 13.5 |
| **6** | 3'-Chlorobenzyl alcohol | DB1701-30m | Method A | 20 | 16.2 | 12.6 | 13.2 |
| **7** | 2'-Chlorobenzyl alcohol | DB1701-30m | Method A | 20 | 15.3 | 12.3 | 14.4 |
| **8** | 4'-Bromobenzyl alcohol | DB1701-30m | Method A | 20 | 18.5 | 14.6 | 15.4 |
| **9** | 3'-Bromobenzyl alcohol | DB1701-30m | Method A | 20 | 18.7 | 14.6 | 15.1 |
| **10** | 2'-Bromobenzyl alcohol | DB1701-30m | Method A | 20 | 17.3 | 14.2 | 16.4 |
| **11** | 4'-Methylbenzyl alcohol | DB1701-30m | Method A | 20 | 13.4 | 11.6 | 12.7 |
| **12** | 3'-Methylbenzyl alcohol | DB1701-30m | Method A | 20 | 13.4 | 11.3 | 12.3 |
| **13** | 2'-Methylbenzyl alcohol | DB1701-30m | Method A | 20 | 13.7 | 11.1 | 11.6 |
| **14** | 4'-Methoxybenzyl alcohol | HP-5 | Method B | 20 | 9.2 | 8.8 | 9.1 |
| **15** | 3',4'-Dimethoxybenzyl alcohol | HP-5 | Method C | 30 | 21.4 | 19.5 | 20.9 |
| **16** | 4-(hydroxymethyl)pyridine | DB1701-30m | Method A | 20 | 15.3 | 9.5 | 9.7 |
| **17** | 3-(hydroxymethyl)pyridine | DB1701-30m | Method A | 20 | 14.8 | 10.3 | 10.9 |
| **18** | Cinnamyl alcohol | HP-5 | Method B | 20 | 9.5 | 9.0 | 9.4 |

For precise determination of conversion, response factors of benzylalcohol, benzylaldehyde, and benzylnitrile were determined. Samples for calibration curves were prepared starting from 50 mM stock solution of respective compound in ethylacetate. The response factors were 68.79 for benzylalcohol, 65.1 for benzylaldehyde and 77.76 for benzylnitrile.

The experiments were performed in two independent duplicate experiments. From obtained values, standard deviations were calculated. Examples 1 and 2 were performed in triplicate and standard deviation was calculated. If necessary, Dean-Dixon's q-test was used to search for outliers at 95% confidence level.

### Example 1: conversion of alcohols with purified enzymes.

To determine the substrate scope of the process, 20 substrates were selected. The process was run for each substrate under the following conditions: GOx (20 µM), Cu²⁺ (50:1 molar ratio calculated to GOx concentration, from 10 mM copper sulphate stock solution), catalase (0.83 µM), the substrate to be tested (10 mM, final concentration) added as a stock solution in DMSO (2% v/v final concentration in reaction); in ammonium formate (0.5 ml, 200 mM pH 9). All reaction components were pipetted into 1.5 ml Eppendorf tube and samples were subsequently shaken at 170 rpm, 20°C for 24 h. After 24 h, the reactions were stopped by addition of KOH (10 M, 70 µl) and the reaction medium was extracted twice with ethylacetate (300 and 350 µl). The combined organic phase was dried over MgSO₄ and conversions were determined via GC-FID. The experiment was performed in duplicate reactions for each substrate and the average value is reported for each reaction. Results are summarized in Table 1 below.

**Table 1. Substrate scope. Absolute conversion of substrates to corresponding nitrile was calculated using 10 mM toluene as internal standard. Relative conversion is calculated in respect to the benzyl alcohol. TON is the turnover number (ratio between the concentration of product formed and GOx catalyst used).**

| | Conversion to nitrile | | |
|---|---|---|---|
| Substrate | Absolute (%) | Relative | TON |
| Benzylalcohol | 45.2 | 100.0 | 226 |
| 4'-Fluorobenzyl alcohol | 28.6 | 63.2 | 143 |
| 3'-Fluorobenzyl alcohol | 19.4 | 43.0 | 97 |
| 2'-Fluorobenzyl alcohol | 43.2 | 95.7 | 216 |
| 4'-Chlorobenzyl alcohol | 24.3 | 53.7 | 121 |
| 3'-Chlorobenzyl alcohol | 9.4 | 20.7 | 47 |
| 2'-Chlorobenzyl alcohol | 43.4 | 96.0 | 217 |
| 4'-Bromobenzyl alcohol | 19.3 | 42.8 | 97 |
| 3'-Bromobenzyl alcohol | 12.9 | 28.6 | 65 |
| 2'-Bromobenzyl alcohol | 28.7 | 63.6 | 144 |
| 4'-Methylbenzyl alcohol | 42.8 | 94.6 | 214 |
| 3'-Methylbenzyl alcohol | 40.9 | 90.5 | 204 |
| 2'-Methylbenzyl alcohol | 13.5 | 29.9 | 68 |
| 4'-Methoxybenzyl alcohol | 23.2 | 51.4 | 116 |
| 3',4'-Dimethoxybenzyl alcohol | 16.7 | 37.0 | 84 |
| *trans*-Cinnamic alcohol | 28.4 | 62.8 | 142 |

As can be seen from Table 1, the process according to the invention results in the conversion of a range of aromatic primary alcohols in the corresponding nitriles.

### Example 2: conversion of alcohols with non-purified cell free extract

To determine whether the use of non-purified crude cell free extract (CFE) is also possible, example 1 repeated. The use of CFE avoids purification steps, saving time and reducing significantly the costs for synthetic applications. CFE was prepared by dissolving the *E. coli* cells harbouring overexpressed GOx in the reaction buffer (1 g of cells per 10 ml of 400 mM Ammonium formate buffer pH 9). Upon the solubilization, cells were sonicated (10 min, 10s on, 10 s off, 45% amp), centrifuged (ca. 35000 g, 15 min, 4°C); the CFE (supernatant) was collected and used for the reaction in the next step. For a reaction in analytical scale (0.5 ml), 0.2 ml of CFE were used (ca. equivalent to supernatant obtained from 0.04 g ml⁻¹ of cells mixture during sonication step). Reaction mixture also contained CuSO₄•₅H₂O (molar ratio with enzyme 50:1), catalase (0.83 µM) and reaction buffer. The reactions were started by the addition of substrate (10 mM as a stock solutions in DMSO, 2% v/v final concentration in reaction) to the reaction mixture in the 1.5 ml Eppendorf tube. Samples were subsequently shaken at 170 rpm, 20°C for 24 h. After 24 h, the reactions were stopped by addition of KOH (10 M, 70 µl) and extracted twice with ethylacetate (300 and 350 µl). The combined organic phase was dried over MgSO₄ and conversions were determined via GC-FID. The experiment was performed in duplicate reactions for each substrate and the average value is reported for each reaction. Results are summarized in Table 2.

**Table 2. Substrate scope - cell free extract. Absolute conversion of substrates to corresponding nitrile was calculated using 10 mM toluene as internal standard. Purity was calculated from the amount of residual intermediate present in the sample.**

| | Conversion (%) | TON |
|---|---|---|
| Benzylalcohol | 65 | 2584 |
| 4'-Fluorobenzyl alcohol | 54 | 2158 |
| 3'-Fluorobenzyl alcohol | 37 | 1483 |
| 2'-Fluorobenzyl alcohol | 70 | 2820 |
| 4'-Chlorobenzyl alcohol | 29 | 1180 |
| 3'-Chlorobenzyl alcohol | 23 | 939 |
| 2'-Chlorobenzyl alcohol | 59 | 2361 |
| 4'-Bromobenzyl alcohol | 16 | 653 |
| 3'-Bromobenzyl alcohol | 22 | 895 |
| 2'-Bromobenzyl alcohol | 39 | 1544 |
| 4'-Methylbenzyl alcohol | 48 | 1917 |
| 3'-Methylbenzyl alcohol | 45 | 1791 |
| 2'-Methylbenzyl alcohol | 24 | 979 |
| 4'-Methoxybenzyl alcohol | 63 | 2518 |
| 3',4'-Dimethoxybenzyl alcohol | 43 | 1726 |
| 4-(Hydroxymethyl)pyridine | 10 | 380 |
| 3-(Hydroxymethyl)pyridine | 55 | 2203 |
| *trans*-Cinnamic alcohol | 10 | 391 |

The results in table 2 show that CFE also results in the formation of nitriles from primary alcohols.

### Example 3: optimisation of the amount of Cu²⁺

GOx possesses a copper metal centre as cofactor. Thus, the enzyme active site must contain the metal centre for catalytic activity. As the metal cofactor may be not tightly bound when the enzyme is applied in solution, the influence of supplementation of catalytic Cu²⁺ was tested. Reaction mixture consisted of Tris-HCI buffer (0.5 mL, 100 mM, pH 8) containing GOx (2.5 µM final concentration, from stock solution in reaction buffer), varying amounts of CuSO₄•5H₂O (0 - 80:1 molar ratio calculated to GOx concentration, from stock solution in reaction buffer) and benzylalcohol as substrate (10 mM). Reactions were run into 1.5 ml Eppendorf tube and shaken at 170 rpm, 30°C in orbital shaker for 40 min. After 40 min, the reactions were stopped by addition of aqueous KOH (10M, 70 µl) and the medium was extracted twice with ethylacetate (300 and 350 µl). The combined organic phase was dried over MgSO₄ and conversions were determined by GC-FID. Experiments were performed in two independent sets of duplicate reactions for each condition (4 samples in total). Results are summarized in Table 3.

**Table 3. Influence of the concentration of Cu²⁺ on the activity of GOx. Cu²⁺/E ratio represents the molar ratio between Cu²⁺ ions and GOx. Reactions in Tris-HCI buffer (100 mM, pH 10), GOx (2.5 µM), benzylalcohol (10 mM), 30°C, 40 min.**

| Cu/E ratio | Relative conversion (%) |
|---|---|
| 0 | 71.8± 9.1 |
| 0.4 | 73.7 ± 8.6 |
| 1 | 58.9 ± 15.0 |
| 2 | 74.9 ± 17.3 |
| 4 | 70.1 ± 16.4 |
| 10 | 75.0 ± 14.3 |
| 20 | 80.2 ± 10.5 |
| 30 | 83.8 ± 15.4 |
| 40 | 90.4 ± 14.5 |
| 60 | 100 ± 8.4 |
| 80 | 97.8 ± 6.4 |

Copper is already present in the enzyme as provided. Table 3 shows that conversion is obtained without adding additional copper. However, the provision of additional copper leads to an increased conversion within the time frame of the experiment.

### Example 4: optimisation of the amount of nitrogen source (ammonium species)

Ammonium ions serve as a nitrogen source for the formation of nitriles. However, high concentrations of NH₄⁺ might have a negative effect on stability of the enzyme. The optimal concentration of ammonium formate buffer was determined in concentration range from 100 mM to 1 M. For each experiment, the enzyme was brought into the buffer at issue by buffer exchange using Vivaspin (Milipore), followed by washing the enzyme with 5 volumes of reaction buffer. The reaction mixture contained GOx (10 µM), Cu²⁺ (50:1 molar ratio calculated to GOx concentration, from 5 mM copper sulphate stock solution), catalase (0.83 µM) and benzylalcohol (10 mM) in ammonium formate (0.5 ml, pH 9) at varied concentration (100 mM - 1 M). All reaction components were pipetted into 1.5 ml Eppendorf tube and shaken in orbital shaker at 170 rpm, 30°C for 24 h. After 24 h, the reactions were stopped by addition of KOH (10 M, 70 µl) and extracted twice with ethylacetate (300 and 350 µl). The combined organic phase was dried over MgSO₄ and conversions were determined via GC-FID. The experiment was performed in two independent sets of duplicate reactions for each condition (4 samples in total). Results are summarized in Table 4.

**Table 4. Influence of the concentration of ammonium/ammonia species on production of benzylnitrile in ammonium formate buffer (pH 9, varied concentration 100 mM -1 M) with GOx (10 µM), Cu²⁺ (1 mM), benzylalcohol (10 mM), catalase (0.83 µM), at 30°C, 24 h.**

| Ammonium species (mM) | Relative conversion (%) |
|---|---|
| 100 | 23.2 ± 0.7 |
| 200 | 55.7 ± 1.6 |
| 400 | 99.5 ± 1.0 |
| 600 | 100.0 ± 1.9 |
| 800 | 86.4 ± 2.0 |
| 1000 | 83.6 ± 1.9 |

From table 4 it can be seen that there is an optimum for the amount of ammonium.

### Example 5: optimisation of the reaction temperature

The effect of reaction temperature was investigated as follows. The reaction mixture contained GOx (20 µM), Cu²⁺ (50:1 molar ratio calculated to GOx concentration, from 5 mM copper sulphate stock solution), catalase (0.83 µM) and benzylalcohol (10 mM) in ammonium formate (0.5 ml, 600 mM, pH 9). All reaction components were pipetted into 1.5 ml Eppendorf tube and shaken in orbital shaker at 170 rpm, for 24 h at the specified reaction temperature. After 24 h, the reactions were stopped by addition of KOH (10 M, 70 µl) and the reaction medium extracted twice with ethylacetate (300 and 350 µl). The combined organic phase was dried over MgSO₄ and conversions were determined via GC-FID. The results are given in Table 5.

**Table 5. Influence of temperature on the production of benzylnitrile in ammonium formate buffer (pH 9, 600 mM) with GOx (20 µM), Cu²⁺ (1 mM), benzylalcohol (10 mM), catalase (0.83 µM), at varying temperature (10 - 50 °C), 24 h.**

| Temperature (°C) | Relative conversion (%) |
|---|---|
| 10 | 31.7 ± 1.0 |
| 20 | 85.7 ± 1.7 |
| 30 | 100.0 ± 0.7 |
| 40 | 74.5 ± 0.3 |
| 50 | 45.2 ± 0.8 |

As can be seen from Table 5, the enzyme used in this example performs best at 30°C. For other enzymes the optimum may be at a different temperature.

### Example 6: influence of the addition of molecular oxygen

In the reactions involving alcohol oxidases, molecular oxygen is often used as final electron acceptor. To investigate whether elevated oxygen level can significantly increase conversion, four conditions were compared. The reaction mixture contained GOx (20 µM), Cu²⁺ (50:1 molar ratio calculated to GOx concentration, from 5 mM copper sulphate stock solution), catalase (0.83 µM) and benzylalcohol (10 mM) in ammonium formate (0.5 ml, 600 mM, pH 9). All reaction components were pipetted into Rotilabo sample vials 2 ml, closed with Screw caps with tiny hole (both Carl Roth GmbH) and PTFE septa (8 mm*0.010) that were pierced with needle (22G1, Becton Dickson) to allow the provision of oxygen gas into the vial during the course of the reaction. Samples were subsequently transferred into a chamber that was pressurized either with 2 bar of pressurized air, 2 bar of pure O₂ or 3 bar of pure O₂ (conditioning with 1 min of flushing). The reactions were shaken in the chamber in an orbital shaker at 170 rpm, 30°C for 24 hours. After the time designated for the reaction, reaction mixtures were transferred into 1.5 ml Eppendorf tube. The reaction was stopped by addition of KOH (10 M, 70 µl) and the reaction mixture was extracted twice with ethylacetate (300 and 350 µl). The combined organic phase was dried over MgSO₄ and conversions were determined via GC-FID. The experiment was performed in two independent sets of duplicate reactions for each condition (4 samples in total). Results are summarized in Table 6.

**Table 6. Influence of O₂ supplementation on production of benzylnitrile in ammonium formate buffer (600 mM, pH 9) with GOx (20 µM), Cu²⁺ (0.5 mM), benzylalcohol (10 mM), catalase (0.83 µM), 30°C, 24 h, 1-2 bar of air or 2-3 bar of O₂. Atm means atmospheric pressure.**

| Absolute O₂ pressure (bar), (gaseous medium) | 24 hours |
|---|---|
| | Relative conversion (%) |
| 1 bar, (air) | 71.0 ± 1.9 |
| 2 bar, (air) | 84.9 ± 2.8 |
| 2 bar (O₂) | 93.9 ± 2.9 |
| 3 bar (O₂) | 100.0 ± 2.2 |

As can be seen from Table 6, the enzyme can tolerate pressure above atmospheric pressure. The optimum of activity is at 2 - 3 bar of pressure. The use of pure molecular oxygen compared to air has only a minor positive influence. However, the use of air is more convenient for this application as air is significantly cheaper than molecular oxygen. For other enzymes, the optimum may be at a different pressure. The use of the enzyme at atmospheric pressure is, however, preferred as no energy is consumed for compression.

### Example 7: influence of pH

The general pH optimum of GOx for the conversion of alcohols into aldehydes was reported to be at pH 7.4 in A. Toftgaard Pedersen, W. R. Birmingham, G. Rehn, S. J. Charnock, N. J. Turner, J. M. Woodley, Organic Process Research & Development 2015, 19, 1580-1589. To determine the optimum pH for the production of nitriles, the following experiments were carried out. Ammonium formate buffer (600 mM) was chosen for the initial study. Five buffer solutions with pH ranging from 8-10 were prepared. The pH was adjusted by addition of either KOH or formic acid. The reaction mixture contained GOx (20 µM), Cu²⁺ (50:1 molar ratio calculated to GOx concentration, from a 10 mM stock solution of copper sulphate in reaction buffer), catalase (17 µM) and benzylalcohol (10 mM) in ammonium formate buffer (0.5 mL, 600 mM) at different pH values. All reaction components were pipetted into 1.5 ml Eppendorf tubes and shaken in orbital shaker at 170 rpm, 30°C for 24 h. After 24 h, the reactions were stopped by addition of KOH (10 M, 70 µl) and the reaction medium was extracted twice with ethylacetate (300 and 350 µl). The combined organic phase was dried over MgSO₄ and conversions were determined via GC-FID. Experiment was performed in two independent sets of duplicate reactions for each condition (4 samples in total). Results are summarized in Table 7.

**Table 7. Influence of pH on the production of benzylnitrile in ammonium formate buffer (600 mM, pH 8-10) with GOx (20 µM), Cu²⁺ (1 mM), benzylalcohol (10 mM), catalase (17 µM), 30°C, 24 h.**

| pH | Relative conversion (%) |
|---|---|
| 8 | 24.5 ± 11.9 |
| 8.5 | 82.5 ± 11.2 |
| 9 | 100 ± 23.0 |
| 9.5 | 74.4 ± 6.2 |
| 10 | 14.1 ± 3.4 |

From Table 7 it can be seen that for this enzyme the optimum pH for nitrile formation is 9. For other enzymes the optimum may be different. In general, a pH optimum in the 7 to 10 pH range is expected.

### Example 8: Presence of catalase

H₂O₂ is produced as secondary product in catalytic cycle of GOx (J. W. Whittaker, Archives of biochemistry and biophysics 2005, 433, 227-239). As H₂O₂ may reduce enzyme activity at certain concentrations, catalase is commonly added in GOx-catalysed reactions in order to convert H₂O₂ to water and oxygen. In many of the experiments described wherein, catalase was used in high excess (17 µM)). To investigate whether lower amounts of catalase may be sufficient to sustain the elevated activity of GOx for longer times the following experiments were carried out. The reaction mixture contained GOx (20 µM), Cu²⁺ (50:1 molar ratio calculated to GOx concentration, from 10 mM copper sulphate stock solution), varied concentration of catalase (0.085 µM - 17 µM) and benzylalcohol (10 mM) in ammonium formate (0.5 mL, 600 mM, pH 9). All reaction components were pipetted into 1.5 ml Eppendorf tube and shaken in orbital shaker at 170 rpm, 30°C for 24 h. After 24 hours the reactions were stopped by addition of KOH (10 M, 70 µl) and the reaction medium was extracted twice with ethylacetate (300 and 350 µl). The combined organic phase was dried over MgSO₄ and conversions were determined via GC-FID. The Experiment was performed in two independent sets of duplicate reactions for each condition (4 samples in total). Results are summarized in Table 8.

**Table 8: Influence of amount of catalase on production of benzylnitrile in ammonium formate buffer (600 mM, pH 9) with GOx (20 µM), Cu²⁺ (1 mM), benzylalcohol (10 mM), varied concentration of catalase (0.085 µM - 17 µM), 30°C, 24 h.**

| Catalase (mg ml⁻¹) | Catalase (µM) | Relative conversion (%) |
|---|---|---|
| 1 | 16.6 | 100 ± 4.8 |
| 0.1 | 1.66 | 96.2 ± 1.0 |
| 0.05 | 0.83 | 97.6 ± 4.2 |
| 0.01 | 0.17 | 85.8 ± 4.3 |
| 0.005 | 0.083 | 79.0 ± 1.2 |

From table 8 it can be seen that the presence of low amounts of catalase are sufficient to obtain meaningful conversion. Example 11 below shows that adequate conversion can also be obtained in the absence of catalase.

### Example 9: verification of the influence of DMSO

Some of the compounds used as starting materials in Examples 1 and 2 are solids at room temperature. Therefore, to ensure full solubility (as well as precise and accurate test at a given concentration) thereof in the reaction mixture they were provided in DMSO. In this example it is investigated whether the presence of DMSO influences the reaction. Three different concentrations of DMSO in the reaction mixture (0, 1, 2 and 5%, v/v) were investigated. The reaction mixture contained GOx (10 µM), Cu²⁺ (50:1 molar ratio calculated to GOx concentration, from 5 mM copper sulphate stock solution), catalase (0.83 µM). Benzylalcohol (final concentration 10 mM) was added as a stock solution in DMSO in a suitable concentration so that the desired concentration of DMSO was obtained in the reaction medium (ammonium formate, 200 mM pH 9, 0.5 ml). All reaction components were pipetted into 1.5 ml Eppendorf tube and shaken in orbital shaker at 170 rpm, 30°C for 24 h. After 24 h, the reactions were stopped by addition of KOH (10 M, 70 µl) and the reaction medium was extracted twice with ethylacetate (300 and 350 µl). The combined organic phase was dried over MgSO₄ and conversions were determined via GC-FID. The experiment was performed in two independent sets of duplicate reactions for each condition (4 samples in total). For each set new enzyme solution was prepared. Results are summarized in Table 9.

**Table 9. Influence of the concentration of DMSO on production of benzylnitrile in ammonium formate buffer (200 mM, pH 9) with GOx (10 µM), Cu²⁺ (0.5 mM), benzylalcohol (10 mM), catalase (0.83 µL), 20°C, 24 h.**

| DMSO (%) | Relative conversion (%) |
|---|---|
| 0 | 94.5 ± 4.5 |
| 1 | 100 ± 5.3 |
| 2 | 96.6 ± 8.7 |
| 5 | 89.3 ± 10.5 |

As can be seen from Table 9 the influence of DMSO on the reaction is limited, making the provision of starting material in aqueous solutions containing DMSO as cosolvent acceptable.

### Example 10: Optimisation of nitrile formation using GOx cell free extract (CFE)

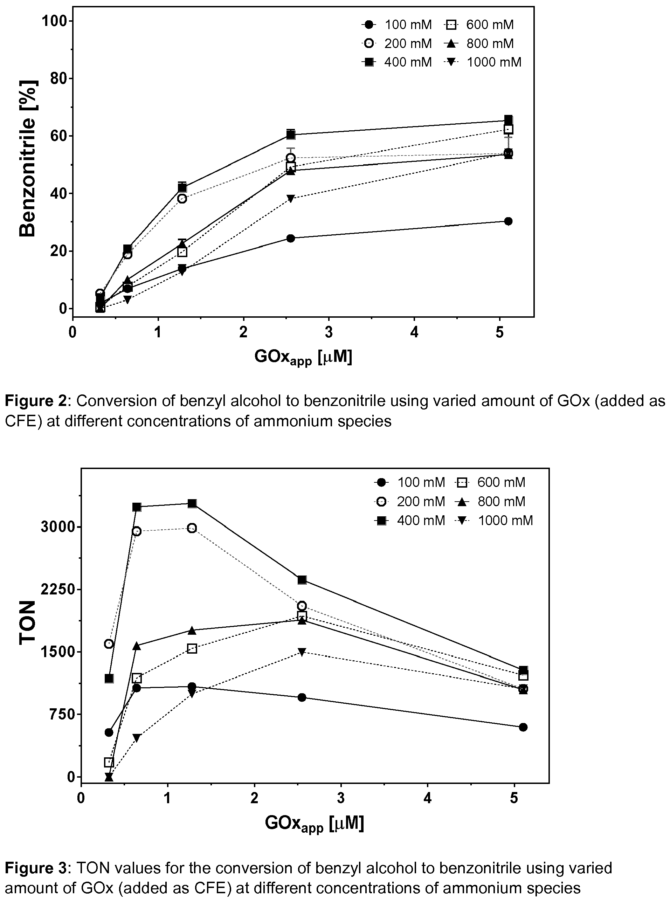

CFE is an attractive material for use in the present invention, as it is more cost-efficient and requires less processing steps than purified enzyme. Therefore, the loading of the GOx (concentration calculated from the purification yields) vs. the concentration of ammonia in the buffer was investigated. GOx crude cell free extract (CFE) was prepared by dissolving the E. coli cells harbouring overexpressed GOx in the reaction buffer (1 g of cells per 10 ml at varied concentration of ammonium formate buffer pH 9). Upon the solubilization, cells were sonicated (10 min, 10 s on, 10 s off, 45% amp), centrifuged (ca. 35000 g, 15 min, 4°C). The CFE (supernatant) was collected and used for performing the biocatalytic reactions. For a reaction in analytical scale (0.5 ml in 1.5 ml Eppendorf tubes), 0.4 ml of CFE were used (ca. equivalent to supernatant obtained from 0.08 g ml-1 of cells mixture during sonication step, equal to 5.10 µM GOx). The reaction mixture also contained CuSO4•5H₂O (1 mM), catalase (0.83 µM) and reaction buffer. The reactions were started by the addition of benzyl alcohol (10 mM, added as DMSO stock solution with a final DMSO content of 2% v/v). Samples were incubated at 170 rpm, 30°C for 24 h. The reactions were stopped by the addition of KOH (10 M, 70 µl) and the reaction medium was extracted twice with ethylacetate. Extracted organic phase was dried over MgSO₄ and conversions were determined via GC-FID (using toluene as internal standard). The efficiency of the CFE catalyzed reaction was visualized in two ways - conversion to benzonitrile (Figure 1) and turnover number, i.e., the number of reactions performed by a single enzyme (Figure 2). Highest TON reaches-3300 (400 mM buffer, 1.28 µM GOx), while highest conversion was 65% (400 mM buffer, 5.1 µM GOx).

From this, the following can be concluded: by using CFE the applicability of the enzyme is significantly improved and the TON reaches the values considered suitable for the industrial application of oxidoreductase enzymes (such as copper-dependent alcohol oxidases from this invention) in the pharmaceutical industry as described in J. Dong et al. Angew. Chem. Int. Ed., 2018, 57, 9238-9261). It is noteworthy that these values of TON have been obtained for a single batch reaction. Recycling and use of the enzyme in subsequent batches or application in flow-reactors will increase the total TON. This technology is available from prior art and already applied for oxidoreductase enzymes as described in K. E. Cassimjee et al., Chem. Commun., 2014, 50, 9134-9137.

### Example 11: influence of the presence of catalase

In order to investigate effect of the addition of catalase to the reaction medium, reactions with three selected substrates were performed as follows: using the purified GOx enzyme in the absence of catalase, using cell free extract (CFE) in the absence of catalase, and using cell free extract (CFE) in the presence of catalase. Benzyl alcohol, 2'-fluorobenzyl alcohol and 2'-chlorobenzyl alcohol were selected as substrates, as high activity was previously determined for the nitrile formation from these substrates in presence of both GOx and catalase.

The reactions with purified GOx enzyme were performed in 0.5 ml of 600 mM Ammonium formate pH 9, containing 20 µM GOx, 1 mM CuSO4•5H2O, and 10 mM substrate added from a DMSO stock solution (2% v/v final concentration of DMSO). The reactions were run for 24 h at 30 °C. Reactions were extracted with 650 µl of EtOAc containing 10 mM toluene as internal standard.

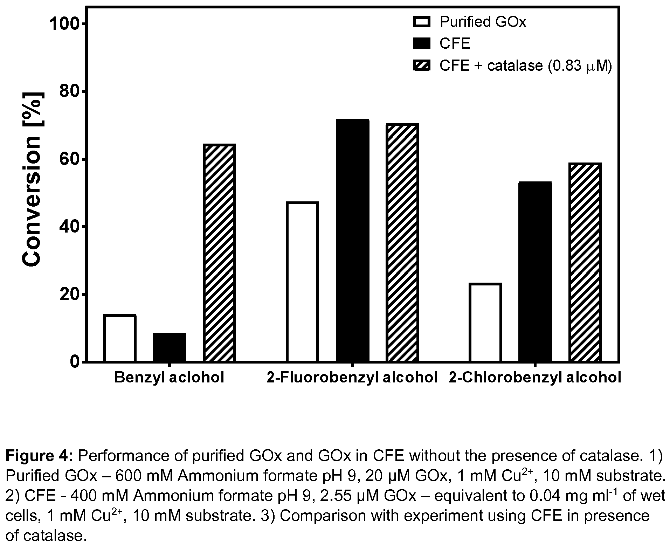

The reactions using GOx as a CFE were performed in 0.5 ml of 400 mM Ammonium formate pH 9, containing 2.55 µM GOx (equal to 0.04 g of wet cells in 1 ml of reaction mixture), 1 mM CuSO4•5H2O, and 10 mM substrate added from a DMSO stock solution (2% v/v final concentration of DMSO). The reactions were run for 24 h at 30 °C. Reactions were extracted in the same manner as for the reactions run with purified GOx. The results (together with comparisons to reactions in presence of catalases) are visualized in Figure 4.

From Figure 4 it can be seen that depending on the nature of the substrate the use of CFE may give a higher efficiency. It should be noted that the CFE has a lower absolute loading of the GOx than the purified enzyme). Moreover, both of the purified GOx and CFE of GOx are able to function without the presence of catalase, offering a possibility for simpler reaction mixture. In conclusion, the addition of a catalase can help to increase the conversion of certain substrates.

### Example 12: Preparative scale experiment

A preparative scale synthesis was performed with 2'-fluorobenzyl alcohol (151 mg, 1.2 mmol) as substrate under the following conditions. 12.5 g of E. coli cells harbouring GOx were solubilized in 125 ml of 200 mM ammonium formate buffer pH 9, sonicated (10 min, 10 s on, 10 s off, 45% amp) and centrifuged (approx. 35000 g, 40 min, 4°C) to obtain a crude cell extract (CFE). The reaction was run in a 500 ml Erlenmayer flask. The reaction mixture of a total of 120 ml in 200 mM ammonium formate pH 9 consisted of CFE previously prepared (96 ml), CuSO4•5H₂O (1 mM) and catalase (0.83 µM). Reaction was started by the addition of 2'-fluorobenzyl alcohol (151 mg, 1.2 mmol). The reaction was incubated at 20°C, 170 rpm for 24 h.

After 24 h, the reaction afforded 94% absolute conversion into the corresponding 2'-fluorobenzonitrile (determined analytically with internal standard). The remaining 6% is accounted for loss of material, in particular the volatile aldehyde intermediate formed during the reaction. The loss of material can be avoided by proper reactor design and engineering as available from existing art in the field. Hence, quantitative conversion could be reached. Finally, after extraction and solvent evaporation, the product was isolated in 76% yield and perfect purity. Thus, further purification procedures are not necessary, which is another great advantage of the present invention.

### Example 13: aldehyde starting material

In the conversion of aromatic alcohol to aromatic nitrile it is believed that the corresponding aromatic aldehyde is an intermediate. Accordingly, aromatic aldehydes can also be used as starting material in the process according to the invention. To illustrate this, the following examples were carried out.

In experiment 1 a reaction mixture was prepared from 0.5 ml of 600 mM Ammonium formate pH 9, containing 20 µM GOx, 1 mM CuSO4*5H2O, and 10 mM benzaldehyde added from a DMSO stock solution (2% v/v final concentration of DMSO). The reaction was run for 24h at 30°C. After 24 hours, the reaction mixture was extracted with 650 µl of EtOAc containing 10 mM toluene as internal standard, and the resulting product was analysed. The result is given in Table 10 below. It can be seen that 47% conversion is obtained.

Experiment 2 was the same as experiment 1, except that 10 mMol H₂O₂ was added to the reaction mixture to mimic the formation of side product in the oxidation of benzylalcohol to benzaldehyde. It can be seen that also in the presence of H₂O₂ significant nitrile formation takes place.

Experiments 3 and 4 were the same as experiments 1 and 2, except that no enzyme was added. It can be seen that no nitrile formation takes place.

Experiments 5 and 6 were the same as experiments 1 and 2, except that instead of enzyme 0.73 mg of bovine albumin was used, which is equivalent to the weight of GOx added in experiments 1 and 2. Again, no nitrile formation takes place.

**Table 10: Aldehyde to nitrile without the catalase. 600 mM Ammonium formate pH 9, 20 µM GOx, 1 mM Cu²⁺, 10 mM substrate, 0 or 10 mM H₂O₂, 0 or 0.73 mg of bovine albumin (equivalent to weight of GOx in reaction).**

| Entry | Albumin | GOx | Cu²⁺ | H₂O₂ | Conversion (%) |
|---|---|---|---|---|---|
| 1 | x | 20 (µM) | 1 (mM) | 0 | 47 |
| 2 | x | 20 (µM) | 1 (mM) | 10 (mM) | 33 |
| 3 | x | x | 1 (mM) | 0 | 0 |
| 4 | x | x | 1 (mM) | 10 (mM) | 0.1 |
| 5 | 0.73 (mg) | x | 1 (mM) | 0 | 0 |
| 6 | 0.73 (mg) | x | 1 (mM) | 10 (mM) | 0.4 |

Overall, this set of experiments conclusively proves that aromatic aldehydes can be converted to nitriles using the process according to the invention. It also shows that the GOx enzyme is necessary to obtain nitrile formation.

## Claims

1. Process for manufacturing aromatic nitriles, wherein a starting material selected from the group of aromatic primary alcohols and aromatic aldehydes is contacted under reaction conditions in a reaction vessel with an inorganic amine compound and molecular oxygen in the presence of a Cu-dependent alcohol oxidase.

2. Process according to claim 1, wherein the starting material is an aromatic primary alcohol of the formula Ar-R-CH₂OH, wherein Ar is an aryl group and R is a covalent bond or an alkylene moiety with 1-20 carbon atoms.

3. Process according to claim 2, wherein Ar is selected from the group of
- substituted or unsubstituted phenyl
- substituted or unsubstituted naphthalyl
- substituted or unsubstituted furanyl
- substituted or unsubstituted pyridinyl
- substituted or unsubstituted pyrazinyl
- substituted or unsubstituted pyrolyl
- substituted or unsubstituted imidazolyl
- substituted or unsubstituted pyrazolyl, and
- substituted or unsubstituted oxazolyl,
preferably a phenyl group, optionally substituted with one or more of halogen, in particular Cl or Br, a C1-C4 alkyl group, or a C1-C4 alkoxy group, or a pyridinyl group, optionally substituted with one or more of halogen, in particular Cl or Br, a C1-C4 alkyl group, or a C1-C4 alkoxy group, in particular unsubstituted phenyl or unsubstituted pyridinyl.

4. Process according to claim 2 or 3, wherein R stands for a covalent bond or an optionally substituted alkylene moiety with 1-10 carbon atoms, preferably a covalent bond or a straight-chain alkylene moiety with 1-4 carbon atoms or a straight-chain alkylene moiety with 2-5 carbon atoms at least one double bond, more preferably a covalent bond or a - CH=CH- moiety.

5. Process according to any one of the preceding claims, wherein the aromatic primary alcohol is selected from the group of benzylalcohol, o-, m-, or p-chlorobenzylalcohol, o-, m-, or p-bromobenzylalcohol, o-, m-, or p-fluorobenzylalcohol, o-, m-, or p-methylbenzylalcohol, o-, m-, or p-methoxybenzylalcohol, dimethoxybenzylalcohol, cinnamylalcohol, and m- and p-pyridylmethanol, in particular benzylalcohol, cinnamylalcohol, and m- and p-pyridylmethanol.

6. Process according to claim 1, wherein the starting material is an aromatic aldehyde of the formula Ar-R-COH wherein Ar is an aryl group, and R is a covalent bond or a C1-C20 alkylene moiety.

7. Process according to any one of the preceding claims wherein the enzyme comprises the aminoacid sequence SEQ ID NO1: 
ASAPIGSAIPRNNWAVTCDSAQSGNECNKAIDGNKDTFWHTFYGANGDPKPPHTYTIDMKT
TQNVNGLSVLPRQDGNQNGWIGRHEVYLSSDGTNWGSPVASGSWFADSTTKYSNFETRP
ARYVRLVAITEANGQPWTSIAEINVFQASSYTAPQPGLGRWGPTIDLPIVPAAAAIEPTSGRV
LMWSSYRNDAFEGSPGGITLTSSWDPSTGIVSDRTVTVTKHDMFCPGISMDGNGQIVVTGG
NDAKKTSLYDSSSDSWIPGPDMQVARGYQSSATMSDGRVFTIGGSFSGGVFEKNGEVYSP
SSKTWTSLPNAKVNPMLTADKQGLYMSDNHAWLFGWKKGSVFQAGPSTAMNWYYTSGS
GDVKSAGKRQSNRGVAPDAMCGNAVMYDAVKGKILTFGGSPDYTDSDATTNAHIITLGEPG
TSPNTVFASNGLYFARTFHTSVVLPDGSTFITGGQRRGIPFEDSTPVFTPEIYVPEQDTFYKQ
NPNSIVRAYHSISLLLPDGRVFNGGGGLCGDCTTNHFDAQIFTPNYLYDSNGNLATRPKITR
TSTQSVKVGGRITISTDSSISKASLIRYGTATHTVNTDQRRIPLTLTNNGGNSYSFQVPSDSG
VALPGYWMLFVMNSAGVPSVASTIRVTQ
 or a sequence having at least 60% sequence identity with SEQ ID NO1, in particular at least 70%, more in particular at least 80%, still more in particular at least 90%.

8. Process according to any one of the preceding claims, wherein the enzyme has an active site equivalent to the active site of the enzyme with the aminoacid sequence SEQ ID NO1, which allows the formation of the active site required to coordinate the Cu cation and to catalyze the reaction of the present invention.

9. Process according to any one of the preceding claims, wherein the enzyme is applied in the form of Cell-free enzyme extract.

10. Process according to any one of the preceding claims, wherein the inorganic amine compound is selected from the group of ammonia (NH₃) and the ammonium ion (NH₄⁺).

11. Process according to any one of the preceding claims, wherein additional Cu²⁺ is provided to the reaction, e.g., in the form or a solution of a copper(II) salt.

12. Process according to any one of the preceding claims, wherein H₂O₂ is removed from the reaction medium during the reaction, preferably by providing catalase to the reaction medium.

13. Process according to any one of the preceding claims, wherein the aromatic nitrile is recovered from the reaction medium, e.g., through extraction with an organic solvent.


SEQUENCE LISTING

<110>  Universiteit van Amsterdam

<120>  Process for the manufacture of nitrile compounds

<130>  EP27631-HS/mc

<140>  EP 18185845
<141>  2018-07-26

<160>  1

<170>  PatentIn version 3.5

<210>  1
<211>  639
<212>  PRT
<213>  Fusarium spp

<400>  1

Ala Ser Ala Pro Ile Gly Ser Ala Ile Pro Arg Asn Asn Trp Ala Val
1               5                   10                  15

Thr Cys Asp Ser Ala Gln Ser Gly Asn Glu Cys Asn Lys Ala Ile Asp
            20                  25                  30

Gly Asn Lys Asp Thr Phe Trp His Thr Phe Tyr Gly Ala Asn Gly Asp
        35                  40                  45

Pro Lys Pro Pro His Thr Tyr Thr Ile Asp Met Lys Thr Thr Gln Asn
    50                  55                  60

Val Asn Gly Leu Ser Val Leu Pro Arg Gln Asp Gly Asn Gln Asn Gly
65                  70                  75                  80

Trp Ile Gly Arg His Glu Val Tyr Leu Ser Ser Asp Gly Thr Asn Trp
                85                  90                  95

Gly Ser Pro Val Ala Ser Gly Ser Trp Phe Ala Asp Ser Thr Thr Lys
            100                 105                 110

Tyr Ser Asn Phe Glu Thr Arg Pro Ala Arg Tyr Val Arg Leu Val Ala
        115                 120                 125

Ile Thr Glu Ala Asn Gly Gln Pro Trp Thr Ser Ile Ala Glu Ile Asn
    130                 135                 140

Val Phe Gln Ala Ser Ser Tyr Thr Ala Pro Gln Pro Gly Leu Gly Arg
145                 150                 155                 160

Trp Gly Pro Thr Ile Asp Leu Pro Ile Val Pro Ala Ala Ala Ala Ile

                165                 170                 175

Glu Pro Thr Ser Gly Arg Val Leu Met Trp Ser Ser Tyr Arg Asn Asp
            180                 185                 190

Ala Phe Glu Gly Ser Pro Gly Gly Ile Thr Leu Thr Ser Ser Trp Asp
        195                 200                 205

Pro Ser Thr Gly Ile Val Ser Asp Arg Thr Val Thr Val Thr Lys His
    210                 215                 220

Asp Met Phe Cys Pro Gly Ile Ser Met Asp Gly Asn Gly Gln Ile Val
225                 230                 235                 240

Val Thr Gly Gly Asn Asp Ala Lys Lys Thr Ser Leu Tyr Asp Ser Ser
                245                 250                 255

Ser Asp Ser Trp Ile Pro Gly Pro Asp Met Gln Val Ala Arg Gly Tyr
            260                 265                 270

Gln Ser Ser Ala Thr Met Ser Asp Gly Arg Val Phe Thr Ile Gly Gly
        275                 280                 285

Ser Phe Ser Gly Gly Val Phe Glu Lys Asn Gly Glu Val Tyr Ser Pro
    290                 295                 300

Ser Ser Lys Thr Trp Thr Ser Leu Pro Asn Ala Lys Val Asn Pro Met
305                 310                 315                 320

Leu Thr Ala Asp Lys Gln Gly Leu Tyr Met Ser Asp Asn His Ala Trp
                325                 330                 335

Leu Phe Gly Trp Lys Lys Gly Ser Val Phe Gln Ala Gly Pro Ser Thr
            340                 345                 350

Ala Met Asn Trp Tyr Tyr Thr Ser Gly Ser Gly Asp Val Lys Ser Ala
        355                 360                 365

Gly Lys Arg Gln Ser Asn Arg Gly Val Ala Pro Asp Ala Met Cys Gly
    370                 375                 380

Asn Ala Val Met Tyr Asp Ala Val Lys Gly Lys Ile Leu Thr Phe Gly
385                 390                 395                 400

Gly Ser Pro Asp Tyr Thr Asp Ser Asp Ala Thr Thr Asn Ala His Ile
                405                 410                 415

Ile Thr Leu Gly Glu Pro Gly Thr Ser Pro Asn Thr Val Phe Ala Ser
            420                 425                 430

Asn Gly Leu Tyr Phe Ala Arg Thr Phe His Thr Ser Val Val Leu Pro
        435                 440                 445

Asp Gly Ser Thr Phe Ile Thr Gly Gly Gln Arg Arg Gly Ile Pro Phe
    450                 455                 460

Glu Asp Ser Thr Pro Val Phe Thr Pro Glu Ile Tyr Val Pro Glu Gln
465                 470                 475                 480

Asp Thr Phe Tyr Lys Gln Asn Pro Asn Ser Ile Val Arg Ala Tyr His
                485                 490                 495

Ser Ile Ser Leu Leu Leu Pro Asp Gly Arg Val Phe Asn Gly Gly Gly
            500                 505                 510

Gly Leu Cys Gly Asp Cys Thr Thr Asn His Phe Asp Ala Gln Ile Phe
        515                 520                 525

Thr Pro Asn Tyr Leu Tyr Asp Ser Asn Gly Asn Leu Ala Thr Arg Pro
    530                 535                 540

Lys Ile Thr Arg Thr Ser Thr Gln Ser Val Lys Val Gly Gly Arg Ile
545                 550                 555                 560

Thr Ile Ser Thr Asp Ser Ser Ile Ser Lys Ala Ser Leu Ile Arg Tyr
                565                 570                 575

Gly Thr Ala Thr His Thr Val Asn Thr Asp Gln Arg Arg Ile Pro Leu
            580                 585                 590

Thr Leu Thr Asn Asn Gly Gly Asn Ser Tyr Ser Phe Gln Val Pro Ser
        595                 600                 605

Asp Ser Gly Val Ala Leu Pro Gly Tyr Trp Met Leu Phe Val Met Asn
    610                 615                 620

Ser Ala Gly Val Pro Ser Val Ala Ser Thr Ile Arg Val Thr Gln
625                 630                 635